# EUROPEAN PATENT APPLICATION

(11) **EP 1 094 311 A2**
(43) Date of publication of application: **25.04.2001**
(21) Application number: 00309219.4
(22) Date of filing: 19.10.2000
(51) Int. Cl.: G01N 1/28, G01N 1/20, B01D 24/00, G01N 33/18

(54) **Fluid sampling**

(30) Priority: 19.10.1999 GB 9924588
(71) Applicant: Snowball, Malcolm Robert, Epping, Essex CM16 6TW (GB)
(72) Inventor: Snowball, Malcolm Robert, Epping, Essex CM16 6TW (GB)
(74) Representative: Evans, Huw David Duncan

(57) **Abstract**

A fluid sampling apparatus comprises a cartridge 11 containing a granular filter media 112. The cartridge 11 is received in a sampling device 23 of the apparatus which comprises an inlet 22 and outlet 21 for the fluid to be sampled and means 14,20 for compacting the granular filter media 112. In use, the fluid to be sampled flows between the inlet 22 and outlet 21 through the compacted granular filter media 112 in the cartridge 11. The cartridge 11 can then be removed from the sampling device 23 to a remote location where the retentate collected on the granular filter media 112 can be removed by backwashing.

Cryptosporidium and other parasites are captured by the compacted granular filter media 112, owing to the compacted media having a pore size which is less than that of the cryptosporidium or other parasites to be detected.

Prior to backwashing, the filter media 112 is allowed to expand and separate, thereby facilitating removal of the retentate therefrom.

## Description

The present invention relates to a fluid sampling and more particularly but not solely to sampling water, in order to detect the presence of cryptosporidium and other parasites.

It is known to sample water for the presence of Cryptosporidium and other parasites. A problem of existing methods is that they are labour intensive and provide poor capture rates of the organisms, usually less than 30%.

An object of this invention is to provide an automatic sampling system for these organisms which reduces the high labour element and provides a high capture sample ready for laboratory testing.

At present, the most widely used system for this type of water sampling is a depth cartridge filter. The water to be tested is passed through a cartridge filter having a 1-micron pore size. Once the required volume of the water sample has passed through the filter, the filter is hygienically bagged and sent to a laboratory for processing. The processing entails elution of the material caught by the filter to flush out the Cryptosporidium or parasite, followed by secondary concentration to produce a sample ready for testing. This is an expensive and time-consuming manual process.

I have now devised a fluid sampling device which alleviates the above-mentioned problems.

In accordance with this invention, there is provided a fluid sampling device comprising a fluid inlet and outlet, a granular filter media arranged to filter fluid flowing from the inlet to the outlet, means for compacting the granular filter media during filtering and means for backwashing the retentate from the filter media, said compacting means allowing the filter media to expand during backwashing.

Also, in accordance with this invention, there is provided a method of sampling a fluid comprising compacting a granular filter media, passing the fluid to be sampled through the compacted filter media, interrupting the fluid flow, removing the compaction of the filter media and backwashing the media to release the retentate therefrom.

Cryptosporidium and other parasites are captured by the compacted granular filter media, owing to the compacted media having a pore size which is less than that of the cryptosporidium or other parasites to be detected.

During backwashing the filter media is allowed to expand and separate, thereby facilitating removal of the retentate therefrom.

Preferably, the media is compacted by a piston or ram. Preferably, the piston or ram is arranged to draw the backwashing fluid through the filter media as it is withdrawn to remove the compaction.

Preferably, the filter media comprises a material with which micro-organisms, their secretory/excretory products, antigens or nucleic acids have an affinity.

The affinity capture material may capture the micro-organisms by means of ionic interactions (charge), non ionic interactions, covalent interactions, hydrophobic/hydrophilic interactions (NB hydrophilic are usually charge mediated) and Ligand-hapten interactions (e.g. antibody/antigen, lectin/sugar, etc.)

All of these mechanisms are characterised by immobilization of the target micro-organism so that they will be isolated from a flow in either a specific or partially specific fashion. These mechanisms can be reversed to release the immobilized micro-organism as and when required.

The capture of the micro-organism is thus by a combination of attachment and size exclusion (filtering): this makes the process both specific and none specific, and of course inherently fail-safe. Once the required volumetric sample has passed through the media, in one embodiment the cartridge is hygienically packed and sent to a laboratory for micro-organism release and analysis.

In other embodiments means are provided to flush the media with a release agent to release the micro-organism or in this case Cryptosporidium into a sample container and therefore provide a laboratory sample for final testing. In these embodiments, after the sample has been produced means are provided to regenerate the media ready to produce the next sample. Means are provided for storing the release agent, the regeneration material and the laboratory sample.

Preferably the system is fully automatic so that the laboratory sample is produced with the minimum of labour content. This system can be used for all micro-organisms including viruses.

Embodiments of this invention will now be described by way of examples only and with reference to the accompanying drawings, in which:
FIGURE 1 is a sectional view through a fluid sampling device in accordance with this invention;
FIGURE 2 is a sectional view through a second embodiment of fluid sampling device in accordance with this invention;
FIGURE 3 is a sectional view through a third embodiment of fluid sampling device in accordance with this invention; and
FIGURE 4 is a sectional view through a fourth embodiment of fluid sampling device in accordance with this invention.

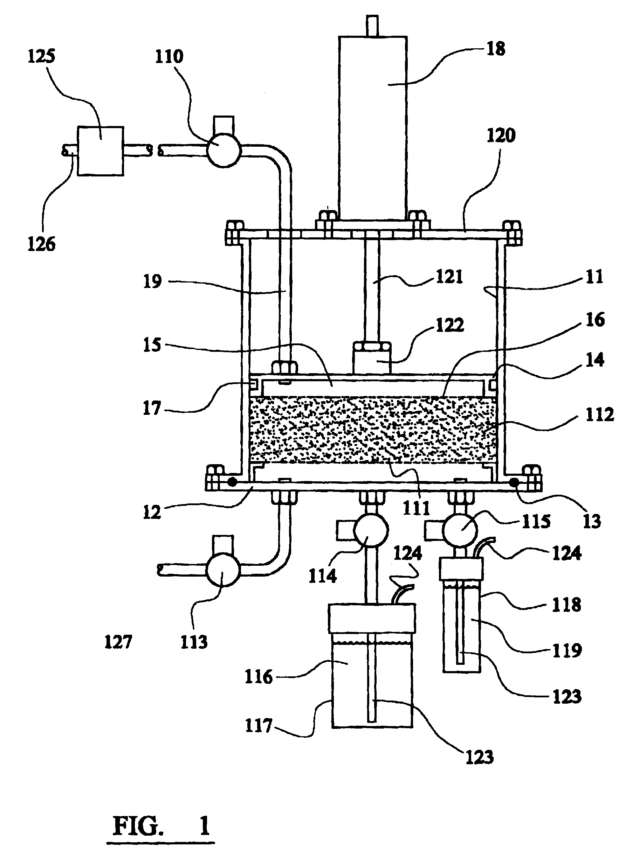

Referring to Figures 1 of the drawings, there is shown a fluid sampling device comprising a watertight cylindrical chamber 11 sealed at one end by plate 12 and seal 13. Positioned in the chamber is a Hollow piston 14. The piston has a cavity 15, which is covered by a mesh 16. Preferably the mesh 16 is attached to the bottom of the piston 14.

The piston is sized to allow it to move up and down the cylindrical chamber 11 and the 'O' ring seal 17 provides a watertight seal preventing any liquid in the chamber from leaking between the piston 14 and the walls of the chamber 11. Attached to the piston 14 is means to move the piston up and down the chamber preferably in the form of a linear electric actuator or pneumatic actuator 18 or some other suitable means.

The electric actuator 18 is supported on a plate 120 and has its shaft 121 attached to the piston 14 via an adjustable coupling 122. This coupling 122 allows easy and accurate setting of the stroke of the electrical actuator this is important for good parasite retention, the media needs to be supported without being packed.

The media 112 is of the specific or semi-specific immobilization type, with its particle size chosen to provide a filter matrix with a pore size suitable for retaining the target micro-organism by size exclusion.

The piston is also fitted to a flexible liquid inlet pipe 19 the other end of which is fixed to the liquid inlet valve 110. Therefor liquid is allowed to flow into the piston cavity 15 and then through the mesh 16. Positioned at the sealed end of the chamber, a short distance from the end plate 12 is a mesh 111, which supports the media 112 placed between it and the mesh 16 fixed to the bottom of the piston. Attached to the end plate 12 are three valves 113, 114 and 115. Valve 113 is the liquid outlet valve. Valve 114 connects the container 117 holding the regeneration material 116, to the chamber 11. Valve 115 connects the sample bottle 118 to the chamber 11.

The sample bottle also contains the release agent 119. Both containers 117 and 118 are fitted with tubes 123, which extend from the valve coupling to substantially the bottom of the container. This tube allows the container to fill and empty. Likewise both containers are fitted with breather tubes 124 to assist in the filling and emptying of the containers. Attached to the chamber is a vibrator mechanism (not shown) which may be mechanical, electrical, ultra-sonic or air agitation to gently break up the media in the chamber during the wash cycle, this ensures that the media does not clump together.

In use, when the electric actuator shaft 121 is fully extended and the piston is at its lowest position, the media between the two meshes 16 and 111 is slightly compressed. A sample bottle containing release agent is attached to solenoid 115 and container 117 is charged with the required amount of regeneration material. Valves 110 and 113 are open and valves 114 and 115 are closed.

The water to be sampled flows through the inlet pipe 126, through the flow meter 125, through valve 110 and into the top of the piston cavity 15 via liquid inlet pipe 19. The main pressure drop in the system is through the media 112 therefor as the water enters the piston cavity 15 its pressure equalizes across the surface area of the media and hence the water flows through the media evenly.

As the water flows through the media, any Cryptosporidium or parasites in the water are immobilized firstly by size exclusion and then through preferential attraction to the media. The water exits via valve 113 and outlet pipe 127. The volume of water passing through the system is monitored by the flow meter 125. The control electronics (not shown) continuously counts the total flow through the system and compares it with a preset volume (sample) size. When the total volume and the preset volume are the same, the system automatically produces a sample in the following manner:

As the water volume equals the preset volume the system goes into a wash cycle. Valves 110 and 113 close stopping further water flow through the system. Valve 115 opens and electric actuator 18 drives piston 14 towards the top of the chamber 11 by shortening its stroke. The piston moves up the chamber 11 drawing the release agent 119 from the sample bottle 118 and through the media. The action of drawing the release agent through the media produces turbulence through the media and creates a washing action.

When the piston has reached its final position in the chamber i.e. the minimum stroke of the electric actuator 18, the actuator reverses and drives the piston 14 towards the bottom of the chamber 11. The release agent is pushed through the media from the opposite direction and back into the sample bottle 117. This cycle is repeated several times. The action of pulling and pushing the release agent through the media together with the gentle vibration from the vibrator on the chamber, produces a washing technique, which detaches the Cryptosporidium or Parasites from the media. At the end of the wash cycle the piston is driven to its lowest position in the chamber 11 i.e., the longest stroke of the electric actuator 18 which pushes the release agent containing the cryptosporidium or Parasites into the sample bottle 118.

Preferably the piston is made to move faster when moving up the chamber than down the chamber: this promotes maximum turbulence with gentle recovery of the micro-organisms. The sample can now be hygienically bagged ready for processing in the laboratory.

Before the system is ready to produce another sample the media must be regenerated. The media is coated with release agent, which must be removed, and the surface of the media restored to its original condition. Going through another wash cycle does this, except that regeneration material is used instead of release agent. Valve 114 is opened and the piston 14 is driven up and down the chamber, drawing the regeneration material 116 back and forth through the media 112 removing the release agent and restoring the surface of the media.

When the wash cycle is completed, the piston is returned to its lowest position in the chamber pushing the regeneration material 116 back into its container 117. At all times the piston 14 remains watertight and airtight with the wall of the chamber via the seal 17 therefor no liquid or air passes the piston 14. The system is ready to take another sample by opening valves 110 and 113.

The control system is relatively simple to achieve for someone skilled in the art and can be accomplished either by Microprocessor, PLC or hard-wired Logic.

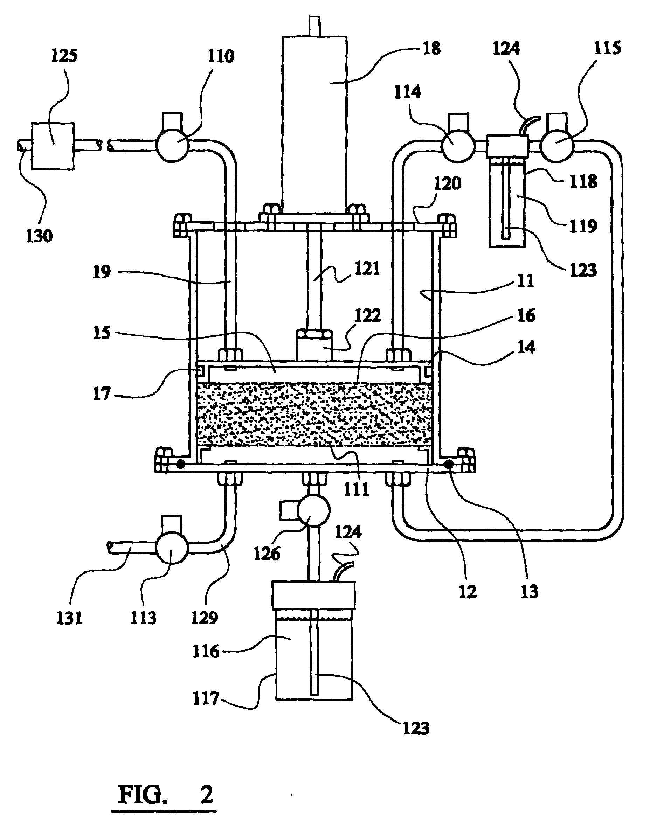

In a second and preferred embodiment of the invention shown in Figure 2, a watertight cylindrical chamber 11 is sealed at one end by plate 12 and seal 13. Positioned in the chamber is a Hollow piston 14. The piston has a cavity 15, which is covered by a mesh 16. Preferably the mesh 16 is attached to the bottom of the piston 14. The piston is sized to allow it to move up and down the cylindrical chamber 11 and the 'O' ring seal 17 provides a watertight seal preventing any liquid in the chamber from leaking between the piston 14 and the walls of the chamber 11. Attached to the piston 14 is means to move the piston up and down the chamber preferably in the form of a linear electric actuator 18.

The electric actuator is supported on a plate 120 and has its shaft 121 attached to the piston 14 via an adjustable coupling 122. This coupling 122 allows easy and accurate setting of the stroke of the electrical actuator this is important for good parasite retention, the media needs to be supported without being packed. The piston is also fitted to a flexible liquid inlet pipe 19 the other end of which is fixed to the liquid inlet valve 110. Therefor liquid is allowed to flow into the piston cavity 15 and then through the mesh 16.

Positioned at the sealed end of the chamber, a short distance from the end plate 12 is a mesh 111, which supports the media 112 placed between it and the mesh 16 fixed to the bottom of the piston. The media is of the specific or semi-specific immobilization type, with its particle size chosen to provide a filter matrix with a pore size suitable for retaining the target micro-organism by size exclusion. Attached to the piston 14 is a flexible pipe 128, which protrudes through the support plate 120 and connects the piston to valve 114, which in turn is connected to the sample bottle 118. The other side of the sample bottle is connected to the end plate 12 via valve 115.

The sample bottle also contains the release agent 119. Also fitted to end plate 12 are the outlet pipe 129 and the chamber containing the regeneration material via valve 126. Both containers 117 and 118 are fitted with tubes 123, which extend from the valve coupling to substantially the bottom of the container. This tube allows the container to fill and empty. Likewise both containers are fitted with breather tubes 124 to assist in the filling and emptying of the containers.

Attached to the chamber is a vibrator mechanism (not shown) which may be mechanical, electrical, Ultra Sonic or air agitation. This gently break up the media in the chamber during the wash cycle, this ensures that the media does not clump together and inhibit recovery of the micro-organisms.

In use, when the electric actuator shaft 121 is fully extended and the piston is at its lowest position, the media between the two meshes 16 and 111 is slightly compressed between the two meshes 16 and 111. The sample bottle 118 containing release agent is in position and fitted between solenoid 114 and solenoid 115. Container 117 is charged with the required amount of regeneration material. Valves 110 and 113 are open and valves 114; 115 and 126 are closed.

The water to be sampled flows through the inlet pipe 130, through the flow meter 125, through valve 110 and into the top of the piston cavity 15 via the flexible liquid inlet pipe 19. The main pressure drop in the system is through the media 112 therefor as the water enters the piston cavity 15 its pressure equalizes across the surface area of the media and hence the water flows through the media evenly. As the water flows through the media any Cryptosporidium or parasites in the water are immobilized firstly by size exclusion and then through preferential attraction to the media. The water exits via outlet pipe 129 and valve 113. The volume of water passing through the system is monitored by the flow meter 125.

The control electronics (not shown) continuously counts the total flow through the system and compares it with a preset volume (sample) size. When the total volume and the preset volume are the same, the system automatically produces a sample in the following manner.

As the water volume equals the preset volume the system goes into its wash cycle. Valves 110 and 113 close stopping further water flow through the system. Valves 114 and 115 open and electric actuator 18 drives piston 14 towards the top of the chamber 11 by shortening its stroke. The piston moves up the chamber 11 drawing the release agent 119 from the sample bottle 118 through valve 115 and through the media, into the chamber 11.

The action of drawing the release agent through the media produces turbulence in the media together with the gentle vibration from the vibrator, creates the washing action. When the piston has reached its final position in the chamber i.e., the minimum stroke of the electric actuator 18, valve 115 closes and valve 114 opens. The actuator reverses and drives the piston 14 towards the bottom of the chamber 11, pushing the release agent through the return pipe 128 and back into the sample bottle via valve 114 and pipe 123. This cycle is repeated several times.

The action of continuously pulling the release agent through the media from bottom to top and the gently vibrating the chamber produces a thorough yet gentle washing action which detaches the Cryptosporidium or Parasites from the media. At the end of the wash cycle valve 114 is closed and the piston is driven to its lowest position in the chamber 11 i.e., the longest stroke of the electric actuator 18. The release agent containing the cryptosporidium or parasites is pushed up the flexible return pipe 128 and into the sample bottle 118 via valve 114. The continuous flushing of the media from the bottom up promotes maximum turbulence, gentle recovery of the micro-organisms and gives a high micro-organism recovery rate, in excess of 80%. When the sample has been taken valve 114 closes. The sample can now be hygienically bagged ready for processing in the laboratory.

Before the system is ready to process another sample the media must be regenerated. The media is coated with release agent, which must be removed, and the surface of the media restored to its original condition. Going through another wash cycle does this, except that regeneration material is used instead of release agent. Valve 126 is opened and the piston 14 is driven up and down the chamber, drawing the regeneration material 116 back and forth through the media 112 and in and out of the container 117, removing the release agent and restoring the surface of the media. When the wash cycle is completed the piston is returned to its lowest position in the chamber pushing the regeneration material 116 back into its container 117 and valve 126 closes. At all times the piston 14 remains watertight and airtight with the wall of the chamber via the seal 17 therefor no liquid or air passes the piston 14. The system is ready to take another sample by opening valves 110 and 113.

The control system is relatively simple to achieve for someone skilled in the art and can be accomplished either by Microprocessor, programmable logic controller (PLC) or hard-wired Logic.

Both fully automatic embodiments are fitted with means to introduce reagents ahead of the units (not shown) to improve the binding action of the media to the micro-organisms.

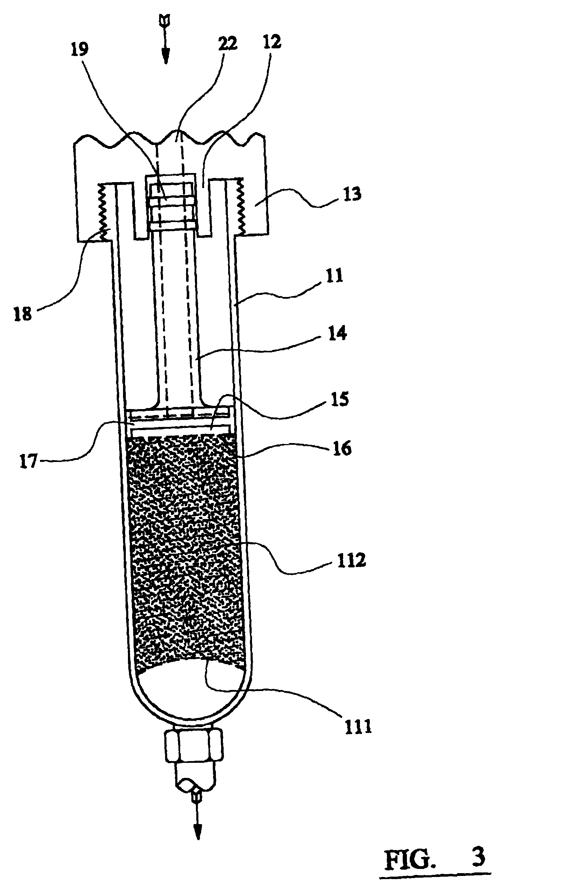

In an embodiment of the invention shown in Figure 3, a watertight cylindrical cartridge 11 is sealed at one end. Positioned in the cartridge is a hollow piston 14. The piston has a cavity 15, which is covered by a mesh 16. Preferably the mesh 16 is attached to the bottom of the piston 14. The piston is sized to allow it to move up and down the cylindrical chamber 11 and the seal 17 provides a watertight seal preventing any liquid in the chamber from leaking between the piston 14 and the walls of the chamber 11. At the top of the cartridge is a screw thread 18, which screws into a holder 13 to support the cartridge 11 and cartridge assembly. The hollow stem of the piston 14 locates into a coupling 12 and the seal 19, as the cartridge 11 is screwed into the holder 13, produces a watertight joint between the coupling 12 and the piston 14. At the other end of the cartridge is an outlet port 21, which is covered by a mesh 111 to constrain filter media 112, contained in the cartridge between meshes 16 & 111, and to prevent it from leaving the cartridge with the liquid. The particle size of the media 112 is chosen to provide a filter matrix with pore size suitable for retaining the target micro-organism by size exclusion.

The liquid to be sampled flows through the piston stem 14 and into the top of the piston cavity 15 and through the media 112. The main pressure drop in the system is through the media 112, therefor as the water enters the piston cavity 15 via the inlet 22, its pressure equalizes across the surface area of the media and hence the water flows through the media evenly. As the water flows through the media any cryptosporidium or parasite in the water is immobilized firstly by size exclusion followed by attachment to the media through preferential attraction. The liquid exits via the outlet port 21 to waste. When the required volumetric water sample has passed through the filter it is hygienically bagged and sent to a laboratory for processing.

At the laboratory the Cryptosporidium can easily be removed from the cartridge by flushing the cartridge media with a release agent under gentle agitation.

For very critical applications the system is made "super safe" by replacing the bottom mesh 111 for a porous membrane or matrix (not shown) whose pore size is much smaller than the size of the target micro-organism. In this case the media also acts as a pre filter for the membrane, preventing it from blocking.

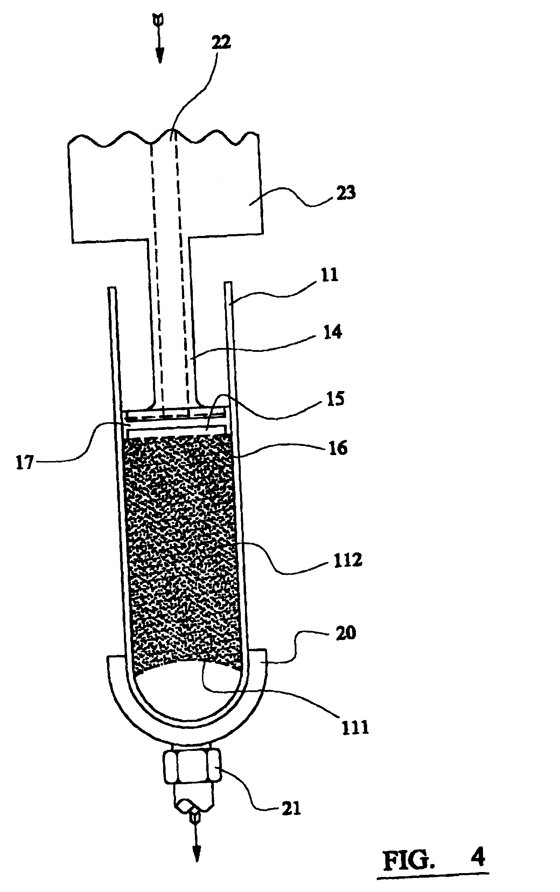

In an alternative embodiment of the invention shown in Figure 4, the piston 14 is fixed and the media 112 is compacted by biasing the cartridge 11 onto the piston 14 either by hand or by means of a biasing mechanism 20 of the apparatus.

## Claims

1. A fluid sampling apparatus comprising a fluid inlet and outlet, a granular filter media arranged to filter fluid flowing from the inlet to the outlet, means for compacting the granular filter media during filtration and means for backwashing retentate from the filter media, said compacting means allowing the filter media to expand during backwashing.

2. A fluid sampling device as claimed in claim 1, in which the media is contained within a chamber, the media being compacted in the chamber by a compacting member within the chamber.

3. A fluid sampling device as claimed in claim 2, in which the media is biased against said compacting member during filtration.

4. A fluid sampling device as claimed in claim 2, in which the apparatus comprises a first portion comprising said inlet, and outlet, a second portion removably mounted to the first portion and comprising said chamber and filter media, and a third remote portion comprising said backwashing means.

5. A fluid sampling device as claimed in any preceding claim, in which the filter media comprises a material arranged to immobilise micro-organisms.

6. A fluid sampling device as claimed in any preceding claim, in which means are provided to flush the filter media with a release agent to release the retentate.

7. A method of sampling a fluid comprising compacting a granular filter media, passing the fluid to be sampled through the compacted filter media, interrupting the fluid flow, removing the compaction of the filter media and backwashing the media to release the retentate therefrom.

8. A method as claimed in claimed 7, comprising compacting the media into a chamber of the apparatus.

9. A method as claimed in claimed 8, comprising biasing the media against said compacting member during filtration.

10. A method as claimed in claims 8 or 9, comprising connecting a cartridge having said chamber and filter media to a first portion of the apparatus, compacting the filter media, passing the fluid to be sampled through the cartridge, removing the compaction of the filter media, removing the cartridge from the first portion of the apparatus to a remote testing portion of the apparatus and backwashing the media to release the retentate therefrom.

11. A method as claimed in any of claims 7 to 10, comprising flushing the filter media with a release agent to release the retentate.
